**Europäisches Patentamt**

**European Patent Office**

**Office européen des brevets**

(19)

(11) **EP 1 061 474 A1**

(12) ## EUROPÄISCHE PATENTANMELDUNG

(43) Veröffentlichungstag:
20.12.2000 Patentblatt 2000/51

(51) Int Cl.⁷: **G06T 7/20**

(21) Anmeldenummer: **99111708.6**

(22) Anmeldetag: **17.06.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(71) Anmelder: **VAMP Verfahren und Apparate der Medizinischen Physik GmbH**
**91096 Möhrendorf (DE)**

(72) Erfinder:
• **Kalender, Willi A., Dr.**
**91096 Kleinseebach (DE)**
• **Kachelriess, Marc, Dr.**
**90409 Nürnberg (DE)**

(54) **Computertomograph mit objektbezogener Bewegungsartefaktreduktion und Extraktion der Objektbewegungsinformation (Kymogramm)**

(57) Die vorliegende Erfindung beschreibt einen Computertomographen mit Spiralabtastung (Einoder Mehrzeilendetektor) der aus den Aufnahmedaten Informationen über die Objektbewegung (Kymogramm) berechnen kann. Kymogramme (im Sinne der kardiologischen Kymographie) können aus beliebiger Projektionsrichtung aus den gemessenen Rohdaten erstellt werden. Diese Information kann zur bewegungsartefaktreduzierenden Bildrekonstruktion genutzt werden und ein Verfahren zur automatischen bewegungsartefaktfreien Bildrekonstruktion wird vorgeschlagen.

Figur 2: Multiplanare Reformation eines Herzens. Das gezeigte Volumen wurde mit einem Standardalgorithmus rekonstruiert. Die Kanten des MPRs zeigen deutliche Bewegungsartefakte, die automatisiert ausgewertet werden können. Die Achse zeigt entlang der z-Richtung und ist wegen $z = d\alpha/2\pi$ und $t = t_{rot}\alpha/2\pi$ gleichzeitig $z$-Achse, $t$-Achse und $\alpha$-Achse. Die eingezeichneten Pfeile zeigen auf Punkte gleicher Herzphase.

EP 1 061 474 A1

## Beschreibung

### Problemstellung, Zielsetzung, Stand der Technik

Die moderne Röntgen-Computer-Tomographie (CT) hat durch neue Scanverfahren und durch Verbesserungen der Technologie ein breites Anwendungsspektrum erreicht. Die Spiral-CT, kontinuierliche Datenakquisition bei gleichzeitigem Patientenvorschub, ermöglicht sehr schnelle Volumenaufnahmen [1; 2]. Um die damit möglichen Volumenaufnahmen weiter zu beschleunigen, wurden zwischenzeitlich Scanner mit mehr als 1 Detektorzeile entwickelt, die weniger als 1 Sekunde pro Umdrehung für die Datenerfassung benötigen. Auf der Basis dieser Fortschritte wird seit einiger Zeit versucht, auch die Bildgebung am Herzen mit Spiral-CT durchzuführen. Es konnte gezeigt werden, daß Bilder trotz der hohen Bewegungsgeschwindigkeit des Herzens in guter Qualität errechnet werden können, wenn Information zur Herzbewegung mittels Elektrokardiogramm (EKG) zur Verfügung steht [3].

Mit Hilfe dieser Ansätze wurden zwar sehr gute Ergebnisse erzielt, es zeigen sich aber auch größere Probleme:

1. Es ist nicht immer einfach möglich, dem EKG exakt eine Bewegungsphase zuzuordnen. Insbesondere muß aufgrund der Reizausbreitung im Herzen berücksichtigt werden, daß bei gegebenem EKG, also gegebener Herzphase, unterschiedliche Abschnitte des Herzmuskels zu unterschiedlichen Zeitpunkten relativ zum RR-Intervall des EKGs die jeweils gewünschte Bewegungsphase durchlaufen.

2. Das Anlegen des EKGs ist mit zusätzlichem Aufwand und mit Zeitverlust in der Untersuchungsvorbereitung verbunden. Die Elektrodenkabel können auch während der Untersuchung störend wirken.

Es wird deshalb gefordert, Bilder retrospektiv aus Spiral-CT-Daten für beliebig wählbare Bewegungsphasen rekonstruieren zu können, ohne dafür eine EKG-Aufzeichnung zu benötigen. Eine Lösung dieses gestellten Problems ist bisher nicht bekannt.

Ferner erscheint es wünschenswert, dem Anwender, hier speziell dem Kardiologen, eine Kymogrammdarstellung zu bieten. Kymogramme sind dem Kardiologen geläufig und können - als Zusatz zu den in der Computertomographie üblichen Schnittbildern - zur Verbesserung der Diagnose beitragen. *(Kymographie, griech. 'Wellenschreibung', das Aufzeichnen von physikalischen und physiologischen Veränderungen als Funkion der Zeit.)*

Zusammenfassend lassen sich folgende Ziele festhalten:

1. Ein Spiral-Computertomograph, der alleine aus den aufgenommenen Spiralmeßdaten (Rohdaten) Informationen über die Objektbewegung extrahiert (unabhängig von Zusatzinformationen wie z.B. EKG). Somit wird ein zusätzlich angelegtes EKG überflüssig und bereits bekannte Algorithmen zur artefaktfreien Darstellung bewegter Objekte bei bekannter Bewegungsfunktion können dennoch angewendet werden.
($\rightarrow$Patentanspruch 1, 5, 8)
2. Eine Methode, die automatisiert, also ohne Anwenderinteraktion, die Fähigkeit besitzt nahezu bewegungsartefaktfreie Bilder eines periodisch (im weiteren Sinne) bewegten Objektes zu erstellen ohne den Umweg über Bewegungsfunktionen zu gehen.
($\rightarrow$Patentanspruch 9)
3. Eine Möglichkeit aus gemessenen Spiralrohdaten Kymogramme zu berechnen.
($\rightarrow$Patentanspruch 2, 3, 4)

### Lösung

Der in Patentanspruch 1 angegebenen Erfindung liegt die Aufgabe zugrunde, für einen Spiral-Computertomographen (Ein- oder Mehrzeiler) die bewegungsphasenkorrelierte Rekonstruktion von CT Bildern ohne Zusatzinformation zu ermöglichen. Damit wird gleichzeitig Information über den Bewegungszustand des Objektes verfügbar.

Die Aufgabe ist erfindungsgemäß gelöst durch die Merkmale des Patentanspruchs.

Die Erfindung ist nachfolgend erläutert. Dabei wird als Beispiel jeweils auf das Herz Bezug genommen, ohne daß damit die Allgemeingültigkeit der Aussagen eingeschränkt werden soll.

### Lösung Ziel 1: Berechnung der Kymogrammfunktion aus den Spiralrohdaten

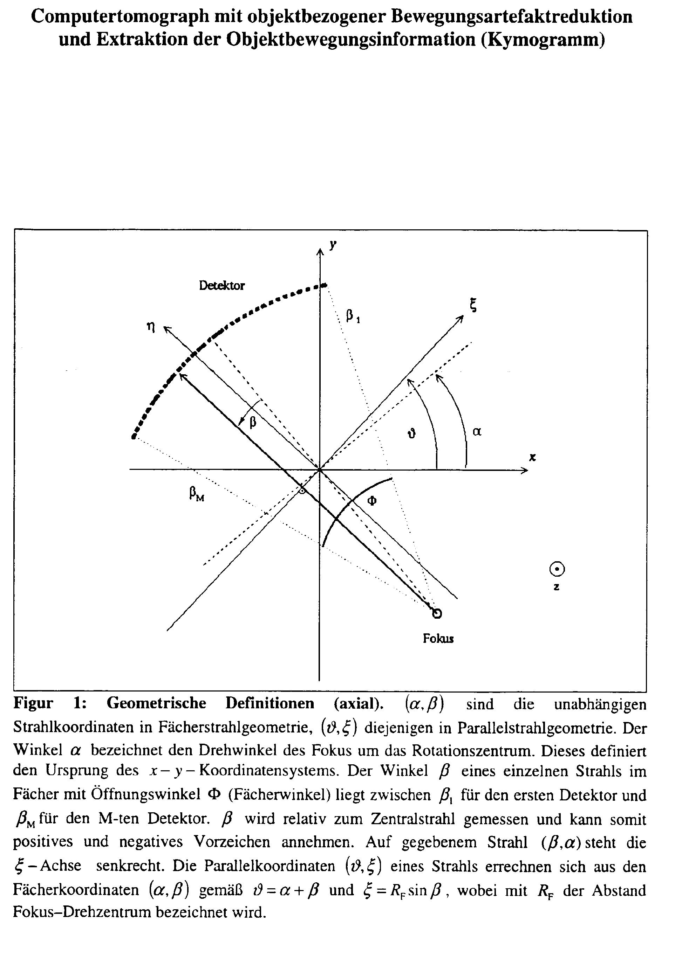

Methode 1, direkte Verwendung der Rohdaten:
Es bezeichne $p(\beta,\alpha,m)$ die gemessenen Spiralrohdaten (Schwächungswerte). Dabei sei $\beta$ der Winkel im Fächer, $\alpha$ der Projektionswinkel und $m$ die Nummer der Detektorzeile (vgl. Figur 1). Die z-Position des Fokus hängt mit

$$z = d \ \alpha/2\pi$$

eindeutig vom Projektionswinkel $\alpha$ ab und tritt deshalb nicht als explizite Variable auf; $d$ sei der Tischvorschub pro 360° Gantryrotation. Die z-Position der Detektorzeile $m$ errechnet sich aus Schichtdicke $S$ (auf das Rotationszentrum umgerechnete Kollimierung einer Detektorzeile) und der Fokusposition zu $z(\alpha, m) = z(\alpha) + S \times (m - (M + 1)/2)$. $M$ bezeichnet hier die Gesamtanzahl der Detektorzeilen und es wurde oBdA angenommen, daß das Detektorarray in z-Richtung auf die Fokusposition zentriert sei.

Der Zeitpunkt $t$ der Messung der Projektion $\alpha$ hängt ebenfalls eindeutig vom Projektionswinkel ab:

$$t = t_{rot} \ \alpha/2\pi,$$

$t_{rot}$ bezeichnet die Umlaufzeit einer 360° Gantryumdrehung.

Um Aussagen über die (unbekannte) Objektbewegung machen zu können, also um ein Kymogramm erstellen zu können, ist es nötig, daß das interessierende Volumen zeitlich überlappend abgetastet wird. Dies wird durch einen Tischvorschub $d < SM$ erreicht, also einen Tischvorschub pro Umdrehung der kleiner ist als die z-Ausdehnung des gesamten Detektorarrays. Das Verhältnis aus Tischvorschub und z-Ausdehnung wird oft auch als Pitch $p$ bezeichnet: $p = d/SM$ . Die Bedingung zur überlappenden Abtastung lautet nun einfach p < 1. Die Anzahl der Rotationen, während derer eine gegebene z-Position abgetastet wird, ist damit $1/p$. Dies entspricht einer Zeitdauer von $t_{rot}/p$. Typische Werte für $t_{rot,}$ liegen beispielsweise zwischen 0.5 s und 1.0 s, typische Werte für den Pitch betragen 0.25 < $p$ < 2. Unter der Voraussetzung, daß die Bewegungsperiode $T$ des Objektes kleiner ist als die Dauer $t_{rot}/p$ der überlappenden Datenaufnahme einer gegebenen z-Position $z_R$ (Rekonstruktionsposition), kann die Bewegungsfunktion mit einer zeitlichen Auflösung von $t_{rot}/2$ wie folgt rekonstruiert werden.

Es sei $p_{\alpha_0} (\xi, \vartheta, z_R)$ ein an der Stelle $z_R$ interpolierter planarer Datensatz in Parallelgeometrie mit maximaler Zeitauflösung, d.h. ein aus 180° Teilumlauf z-interpolierter Datensatz. $\xi$ bezeichnet den Abstand eines Strahls in Parallelgeometrie zum Rotationszentrum, $\vartheta$ bezeichnet dessen Rotationswinkel, z.B. relativ zur x-Achse (die Parallelkoordinaten $\xi$ und $\vartheta$ entsprechen den Fächerkoordinaten $\beta$ und $\alpha$, vgl. Figur 1). Der Index $\alpha_0$ zeigt an, um welchen Projektionswinkel der gemessenen Spiraldaten herum die Interpolation zentriert wurde. Der Wertebereich für $\vartheta$ liegt somit zwischen $\vartheta_{min}$ = $\alpha_0$ - $\pi/2$ und $\vartheta_{min} = \alpha_0 + \pi/2$. Der Parallelwinkel $\vartheta$ überstreicht also in jedem Fall ein 180° Intervall und somit ist der interpolierte Datensatz $p_{\alpha_0} (\xi, \vartheta, z_R)$ vollständig und rekonstruierbar. Da, wie oben erwähnt, die Datenaufnahme überlappend erfolgt existiert zu jeder Rekonstruktionsposition $z_R$ ein ganzes Intervall von Projektionswinkeln $\alpha_0$, um die die z-Interpolation zentriert werden kann: $\alpha_0 \in [\alpha_{0,min}, \alpha_{0,max}]$. Die Menge $\{p_{\alpha_0} (\xi, \vartheta, z_R)| \ \alpha_0 \in [\alpha_{0,min}, \alpha_{0,max}]\}$ aller an der Stelle $z_R$ interpolierbaren 180° Datensätze repräsentiert somit das vollständige Objekt zu allen möglichen Zeitpunkten während der Bewegungsperiode, denn der Winkel $\alpha_0$ entspricht eindeutig einem Zeitpunkt $t_0$ an dem die Projektion $\alpha_0$ gescannt wurde.

Unter Ausnützung der Symmetrie $p_{\alpha_0} (\xi, \vartheta, zR) = p_{\alpha_0} (-\xi, \vartheta + \pi, Z_R)$ der Parallelprojektionen läßt sich für alle interpolierten Datensätze $p_{\alpha_0} (\xi, \vartheta, z_R)$ der Wertebereich der $\vartheta$ von $[\alpha_0 - \pi/2, \alpha_0 + \pi/2]$ auf den Bereich $[0, \pi)$ abbilden und somit die Rohdaten für unterschiedliche $\alpha_0$ miteinander vergleichen. Deshalb wird im folgenden davon ausgegangen, daß die $\vartheta$ implizit im Intervall $[0, \pi)$ enthalten sind. Eine geeignete Funktion, die den Bewegungsablauf des Objekts zum Zeitpunkt $t_0$ (als Funktion von $\alpha_0$) angibt lautet zum Beispiel

$$K(\alpha_0) := \int d\xi \, d\vartheta \left| \frac{\partial}{\partial \alpha_0} p_{\alpha_0}(\xi, \vartheta, z_R) \right|^q w(\xi).$$

Die partielle Ableitung nach der Position $\alpha_0$ entspricht der zeitlichen Ableitung der Objektfunktion an der betrachteten z-Position. Die Ausintegration der Variablen $(\xi, \vartheta)$ reduziert das Kymogramm auf ein Skalar. Dabei kann mit dem Parameter q die Ableitung gewichtet werden. Mit der (symmetrischen) Gewichungsfunktion $w(\xi) = w(-\xi)$ besteht die Möglichkeit beispielsweise Strahlen in der Nähe des Rotationszentrums höher zu gewichten als Strahlen die weit vom Zentrum entfernt liegen (z.B. liegt das Herz, und somit der Hauptanteil der Bewegung nahe des Rotationszentrums).

Diskret kann die partielle Ableitung als Differenzenquotient implementiert werden:

$$K(\alpha_0) := \int d\xi \, d\vartheta \left| \frac{p_{\alpha_0 + \Delta\alpha}(\xi, \vartheta, z_R) - P_{\alpha_0}(\xi, \vartheta, z_R)}{\Delta\alpha} \right|^q w(\xi).$$

Die Kymogrammfunktion $K(\alpha_0)$ zeigt somit Phasen schneller und Phasen langsamer Bewegung auf. Minima von $K(\alpha_0)$ ensprechen den Zeitpunkten $t_0 = t_{rot}\,\alpha_0/2\pi$ niedrigster Herzbewegung, Maxima denjenigen höchster Bewegung.

$K(\alpha_0)$ kann analog zu anderen Bewegungsfunktionen - wie beispielsweise das während des Scans aufgezeichnete EKG-Signal - ausgewertet werden und speziellen Bildrekonstruktionsalgorithmen (z.B. 180°CD [3]) zur bewegungsartefaktreduzierten Rekonstruktion zugeführt werden. Damit besteht die Möglichkeit ohne zusätzliches EKG und ohne Interaktion durch den Anwender bewegungskorrelierte Rekonstruktionen durchzuführen.

Methode 2, Verwendung einer oder mehrerer multiplanarer Reformationen (MPR):

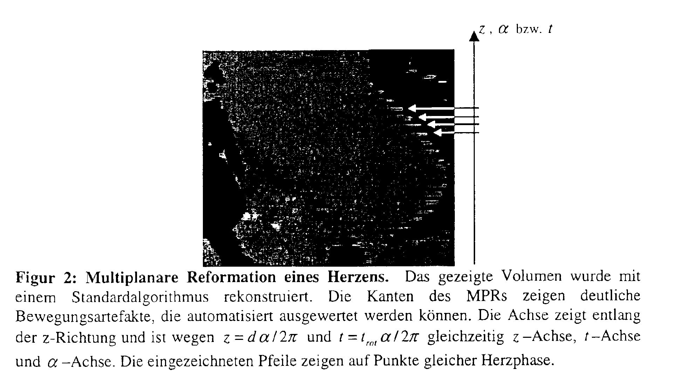

Alternativ zu oben angegebener Methode kann der Umweg über ein mit Standardalgorithmen rekonstruiertes Volumen gegangen werden. Dazu wird der gesamte Spiraldatensatz mit einem Rekonstruktionsalgorithmus rekonstruiert, der nicht bewegungskorreliert arbeitet (Standard z-Interpolation). Im rekonstruierten Volumen $f(x,y,z)$ spiegelt sich die Objektbewegung in Form von Bewegungsartefakten deutlich wieder. Insbesondere in z-Richtung sind die rekonstruierten Schichten $f(x,y,z_R)$ aufgrund der zu aufeinanderfolgenden z-Positionen $z_R$ unterschiedlichen beitragenden Zeitintervalle inkonsistent. Besonders deutlich werden diese Bewegungsartefakte in einer multiplanaren Reformation des Volumens (Darstellung des Volumens entlang einer 2D Ebene), die die z-Achse enthalten oder parallel dazu verlaufen kann. Beispielsweise ist $f(x,0,z)$ ein MPR in dem die x- und z-Achse sowie der Koordinatenursprung enthalten ist. An den Kanten des bewegten Objektes sind nun deutlich die Bewegungsphasen als Sprünge in den Objektkanten unterscheidbar (Figur 2). Diese können automatisch detektiert werden, z.B. über ein Edge-Enhancement Filter in z-Richtung. Die zugehörigen z-Positionen entsprechen direkt einem Zeitpunkt $t$ sowie einer Projektionsnummer $\alpha$ denn $z = d\alpha/2\pi$ und $t = t_{rot}\,\alpha/2\pi$ (s.o.). Damit existiert eine eindeutige Zuordnung der Bewegungsphasen zu den Projektionsnummem und es können, analog zu Methode 1, spezielle Rekonstruktionsalgorithmen (s.o.) eine phasenkorrelierte und artefaktarme Rekonstruktion durchführen.

**Lösung Ziel 2: Automatisierte Bewegungsartefaktreduktion**

Zur bewegungsartefaktarmen Bildrekonstruktion besteht neben dem Umweg über eine Kymogrammfunktion (s.o.) die Möglichkeit aufeinanderfolgende, zu rekonstruierende Schichten, so zu wählen, daß zwischen ihnen möglichst wenig Unterschiede herrschen. Dies ist unter der Annahme gerechtfertigt, daß sich das abzubildende Objekt in z-Richung deutlich weniger verändert als durch seine Bewegung.

Im folgenden sei mit $\Delta z_R$ der Abstand zweier benachbarter zu rekonstruierenden Schichten bezeichnet. Analog zu dem im obigen Abschnitt gesagten, besteht auch hier die Möglichkeit bei gegebener z-Position zu unterschiedlichen Zeitpunkten zu rekonstruieren. Da die Rekonstruktion einer z-interpolierten Schicht ein linearer Prozess ist, brauchen nicht unterschiedliche Bilder miteinander verglichen werden sondern es reicht aus, die interpolierten Rohdatensätze $p_{\alpha_0}(\xi,\vartheta,z_R)$ und $p_{\alpha_0+\Delta\alpha}(\xi,\vartheta,z_R+\Delta z_R)$ möglichst ähnlich zu wählen. Der Parameter $\Delta\alpha$ steht für den Unterschied in den Zeitschwerpunkten beider interpolierten Rohdatensätze. Die Optimierung bezüglich $\Delta\alpha$ geschieht wie folgt

$$\arg\min_{\Delta\alpha}\int d\xi\,d\vartheta\left|p_{\alpha_0}(\xi,\vartheta,z_R)-p_{\alpha_0+\Delta\alpha}(\xi,\vartheta,z_R+\Delta z_R)\right|^q w(\xi)$$

die Funktion des Gewichtes $w(\xi)$ sowie des Parameters $q$ entspricht derjenigen aus dem vorangehenden Abschnitt. Der Bereich in dem $\Delta\alpha$ variiert werden kann, ist durch den an der z-Position $z_R+\Delta z_R$ erlaubten Zeitbereich beschränkt. Die Vorgehensweise ist wie folgt:

Die erste zu rekonstruierende Schicht wird mit willkürlicher Wahl von $\alpha_0$ durchgeführt. Jede weitere z-Interpolation geschieht durch die Optimierung des Zeitinkrements $\Delta\alpha$ gemäß obiger Minimierungsgleichung. Optimiert wird jeweils auf die zuletzt rekonstruierte, also auf die vorangehende benachbarte Schicht. Es kann gezeigt werden, daß die Rekonstruktion gegen eine Phase <u>langsamer</u> Objektbewegung konvergiert. Damit steht ein Algorithmus zur Verfügung, der beliebig bewegte Objekte automatisch in der Phase der geringsten Bewegung rekonstruiert. Im Gegensatz zur artefaktreduzierten kymogrammbasierten Rekonstruktion (vorangehender Abschnitt) ist eine Wahl der Bewegungsphase nicht möglich, da das Verfahren gegen einen festen Punkt konvergiert.

**Lösung Ziel 3: Kymogrammberechnung aus gemessenen Spiralrohdaten**

Eine weitere Lösung des Problems kann in folgender Weise erreicht werden. Aus dem SpiralDatensatz können Projektionsbilder (Schattenbilder) in Fächerkoordinaten, entsprechend den bei CT üblichen Übersichtsaufnahmen (Topogramme), oder in Parallelkoordinaten in beliebiger Projektionsrichtung errechnet werden [4]. Bei überlappender Datenaufnahme besteht nun die Möglichkeit, Daten an gleicher z-Position zu unterschiedlichen Zeitpunkten zu sammeln. Damit ergibt sich am Herzen ein aus der Kardiologie bekanntes Bewegungsbild, das Kymogramm: An der in zeitlicher Reihenfolge gewonnenen einzelnen Streifenprojektion läßt sich die Herzbewegung nachvollziehen. Insbesondere ist auf den Bewegungskonturen der Zeitpunkt der größten Ausdehnung des Herzmuskels (Diastole) und der der stärksten Kontraktion (Systole) abzulesen. Diese Information zum Bewegungsablauf ist entlang der z-Achse (Körperlängsachse) gegeben und ermöglicht somit die Zuordnung der Herzbewegung getrennt für jeden Herzabschnitt. Die Darstellung der gemäß o.g. Patentschrift errechneten und wegen der Überlappung der Datenaufnahme zu verschiedenen Zeitpunkten zur Verfügung stehenden Übersichtsaufnahmen findet analog zur Darstellung der mittels Kymogrammoption (folgender Abschnitt) gemessenen Daten statt.

**Literatur**

[1] Kalender W A, Seissler W, Klotz E, Vock P. Spiral volumetric CT with single-breathhold technique, continuous transport, and continuous scanner rotation. Radiology 1990; 176 (1): 181-183

[2] Kalender W A, Kachelrieß M, Wohlrab J. Grundlagen der Spiral-CT: I. Prinzipien von Aufnahme und Bildrekonstruktion. Z. Med. Phys. 1997; 7: 231-240

[3] Kachelrieß M, Kalender W A. ECG-correlated Image Reconstruction from Subsecond Spiral CT Scans of the Heart. Medical Physics 1998; 25 (12): 2417-2431

[4] Eckert R, Kalender W A (1992). Patentschrift DE 41 03 588 C 1. Bundesrepublik Deutschland

**Patentansprüche**

1. Computertomograph mit Ein- oder Mehrzeilendetektor, der Spiral-CT-Aufnahmedaten zur Verfügung stellt aus denen Information über Objektbewegung, Objektbewegungskonturen (Kymogramme) sowie Objektbewegungsphasen ohne Zusatzinformation (wie z.B. EKG oder spirometrisch gemessene Atemlage) gewonnen werden können.

2. Computertomograph nach Anspruch 1, bei dem aus den Aufnahmedaten Übersichtsaufnahmen (Kymogramme) in beliebiger Projektionsrichtung berechnet werden können.

3. Computertomograph nach Anspruch 1, bei dem aus den Aufnahmedaten Übersichtsaufnahmen von Bewegungen (Kymogramme) für beliebige Phasen der Objektbewegung berechnet werden können.

4. Computertomograph nach Anspruch 2 und 3 bei dem die Übersichtsaufnahmen sowohl aus beliebiger Projektionsrichtung als auch zu beliebigen Zeitpunkten (Bewegungsphasen) erstellt werden können.

5. Computertomograph nach Anspruch 2, 3 und 4, bei dem aus den Kymogrammen die Herzphasenbewegung der Zeitskala und somit den Projektionsnummem zugeordnet werden kann und diese Information zur artefaktreduzierten Bildrekonstruktion (z-Interpolation oder Teilscanrekonstruktion) genutzt wird.

6. Computertomograph nach Anspruch 1, bei dem die Objektbewegungsinformation aus einer bewegungsartefaktbehafteten Rekonstruktion mittels multiplanarer Reformationen des Bildvolumens (MPR) extrahiert wird.

7. Computertomograph nach Anspruch 6, der die aus einer MPR berechnete Bewegungsfunktion zur artefaktreduzierten Spiralinterpolation nutzt.

8. Computertomograph nach Anspruch 1, bei dem für beliebige z-Positionen $z_R$ sowie beliebige Zeitpunkte $t_0$ eine Bewegungsfunktion $K(t_0, z_R)$, die sogenannte Kymogrammfunktion, aus den gemessenen Projektionsdaten (Rohdaten) abgeleitet werden kann.

9. Computertomograph nach Anspruch 1, bei dem ein periodisch bewegtes Objekt nach der Formel

$$\underset{\Delta\alpha}{\arg\min}\int d\xi\, d\vartheta \left| p_{\alpha_0}(\xi,\vartheta,z_R) - p_{\alpha_0+\Delta\alpha}(\xi,\vartheta,z_R+\Delta z_R) \right|^q w(\xi)$$

in seiner bewegungsärmsten Phase z-interpoliert wird. Die Methode ist vollständig automatisiert und auch auf andere Bereiche als das Herz anwendbar.

# Computertomograph mit objektbezogener Bewegungsartefaktreduktion und Extraktion der Objektbewegungsinformation (Kymogramm)

**Figur 1: Geometrische Definitionen (axial).** $(\alpha, \beta)$ sind die unabhängigen Strahlkoordinaten in Fächerstrahlgeometrie, $(\vartheta, \xi)$ diejenigen in Parallelstrahlgeometrie. Der Winkel $\alpha$ bezeichnet den Drehwinkel des Fokus um das Rotationszentrum. Dieses definiert den Ursprung des $x - y -$ Koordinatensystems. Der Winkel $\beta$ eines einzelnen Strahls im Fächer mit Öffnungswinkel $\Phi$ (Fächerwinkel) liegt zwischen $\beta_1$ für den ersten Detektor und $\beta_M$ für den M-ten Detektor. $\beta$ wird relativ zum Zentralstrahl gemessen und kann somit positives und negatives Vorzeichen annehmen. Auf gegebenem Strahl $(\beta, \alpha)$ steht die $\xi -$ Achse senkrecht. Die Parallelkoordinaten $(\vartheta, \xi)$ eines Strahls errechnen sich aus den Fächerkoordinaten $(\alpha, \beta)$ gemäß $\vartheta = \alpha + \beta$ und $\xi = R_F \sin \beta$, wobei mit $R_F$ der Abstand Fokus–Drehzentrum bezeichnet wird.

$z$ , $\alpha$ bzw. $t$

**Figur 2: Multiplanare Reformation eines Herzens.** Das gezeigte Volumen wurde mit einem Standardalgorithmus rekonstruiert. Die Kanten des MPRs zeigen deutliche Bewegungsartefakte, die automatisiert ausgewertet werden können. Die Achse zeigt entlang der z-Richtung und ist wegen $z = d\,\alpha/2\pi$ und $t = t_{rot}\,\alpha/2\pi$ gleichzeitig $z$–Achse, $t$–Achse und $\alpha$–Achse. Die eingezeichneten Pfeile zeigen auf Punkte gleicher Herzphase.

EP 1 061 474 A1

## Europäisches Patentamt
## EUROPÄISCHER RECHERCHENBERICHT

Nummer der Anmeldung

EP 99 11 1708

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | WANG G ET AL: "PRELIMINARY STUDY ON HELICAL CT ALGORITHMS FOR PATIENT MOTION ESTIMATION AND COMPENSATION" IEEE TRANSACTIONS ON MEDICAL IMAGING,US,IEEE INC. NEW YORK, Bd. 14, Nr. 2, Seite 205-211 XP000520932 ISSN: 0278-0062 * Zusammenfassung * | 1-5,8 | G06T7/20 |
| Y | EIHO S ET AL: "Automatic processing of cineangiographic images of left ventricle" PROCEEDINGS OF THE 4TH INTERNATIONAL JOINT CONFERENCE ON PATTERN RECOGNITION, KYOTO, JAPAN, 7-10 NOV. 1978, Seiten 740-742, XP002122709 1978, Kyoto, Japan, Kyoto, Univ, Japan * Zusammenfassung; Abbildungen 5,6 * | 1-5,8 | |
| A | HSIEH J: "A GENERAL APPROACH TO THE RECONSTRUCTION OF X-RAY HELICAL COMPUTED TOMOGRAPHY" MEDICAL PHYSICS,US,AMERICAN INSTITUTE OF PHYSICS. NEW YORK, Bd. 23, Nr. 2, Seite 221-229 XP000555908 ISSN: 0094-2405 * Seite 221, rechte Spalte, Zeile 3 - Zeile 7 * | 2-4 | RECHERCHIERTE SACHGEBIETE (Int.Cl.7)<br><br>G06T |

Der vorliegende Recherchenbericht wurde für alle Patentansprüche erstellt

| Recherchenort | Abschlußdatum der Recherche | Prüfer |
|---|---|---|
| BERLIN | 15. November 1999 | Bouchaâla, N |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
............................................................................
& : Mitglied der gleichen Patentfamilie, übereinstimmendes Dokument

EPO FORM 1503 03.82 (P04C03)

9